# DEMANDE DE BREVET EUROPEEN

(11) **EP 1 158 448 A1**
(43) Date de publication de la demande: **28.11.2001**
(21) Numéro de dépôt: 00401407.2
(22) Date de dépôt: 22.05.2000
(51) Int. Cl.: G06F 19/00, G06F 1/00, G06F 17/30

(54) **Serveur d'informations médicales**

(71) Demandeur: Descat, Paul-Henri, 94350 Villers sur Marne (FR); Pucci, Daniel, 78150 Le Chesnay (FR)
(72) Inventeur: Descat, Paul-Henri, 94350 Villers sur Marne (FR); Pucci, Daniel, 78150 Le Chesnay (FR)
(74) Mandataire: Cabinet Hirsch

(57) **Abrégé**

L'invention concerne un serveur (12) de données, comprenant pour un adhérent une partie protégée (14) qui contient le dossier médical de l'adhérent. La partie protégée peut être accédée par l'adhérent en lecture d'une part et en écriture sans droit de modifications des données existantes, d'autre part. Elle peut être accédée par une première classe d'utilisateurs en lecture et en écriture; cette classe d'utilisateurs comprend typiquement les médecins ou praticiens dont l'activité rend utile la consultation du dossier médical. Enfin, la partie protégée peut être accédée par une deuxième classe d'utilisateurs, en lecture uniquement des données non-nominatives; la deuxième classe d'utilisateur peut comprendre les laboratoires, les pouvoirs publics ou les assureurs, qui sont intéressés à des données statistiques sur les produits prescrits.

On assure ainsi simplement un suivi complet des adhérents sur le plan médical.

## Description

L'invention concerne la gestion des données médicales et l'accès à ces données, et plus spécifiquement l'accès par le biais d'un réseau à des données médicales.

Le développement du réseau Internet assure aujourd'hui un accès instantané ou quasi-instantané à des sources d'information multiples. Par ailleurs, sur le plan financier, les moyens de paiement par carte de crédit permettent un paiement simple et sécurisé, quasiment universellement disponible.

Dans le domaine de la santé, le retard par rapport à ces évolutions technologiques est patent. Les informations médicales concernant un individu sont dispersées entre les différents praticiens qui ont eu à le soigner, les différents laboratoires ayant effectué les analyses le concernant ou les différents hôpitaux où il a pu consulter, ou encore les pharmacies ayant délivré les médicaments prescrits (ou substitué un générique). Il existe donc un problème de centralisation des informations médicales concernant un individu donné. L'intérêt de la centralisation de telles information est de permettre un meilleur traitement, et d'éviter tout doublonnage dans le diagnostic, dans les analyses ou dans le traitement. Un autre intérêt est de simplifier pour le patient comme pour le corps médical la gestion des informations, comme par exemple la mise à jour des vaccinations. Les solutions existantes comme le carnet de santé se sont avérées largement inefficaces; des solutions comme la carte Vitale mise en place en France - carte à puce sécurisée contenant des éléments du dossier médical de son porteur - sont lourdes à mettre en oeuvre; elles sont aussi d'une capacité limitée par les capacités des cartes à puce; enfin, elles ne permettent pas à un individu d'accéder au contenu de son propre dossier médical, ce vers quoi s'achemine la réglementation française.

Se pose par ailleurs dans le domaine de la santé la question de la statistique de prescription, et des pratiques de prescription. Il existe un besoin de connaître plus précisément les habitudes de prescription du corps médical, à des fins économiques, mais aussi à des fins techniques d'évaluation statistique des effets des actes médicaux en général et des médicaments en particulier.

Enfin, se pose encore dans le domaine de la santé la question de la comparaison des prestations disponibles, tant sur le plan médical, que sur le plan des prestations annexes - prestations et services hôteliers et d'accueil des patients par exemple.

L'invention propose une solution à ces différents problèmes. Pour l'utilisateur, elle permet d'avoir en permanence son dossier médical à sa portée, de choisir sa médecine en connaissance de cause. Dans certains modes de réalisation, elle lui permet encore de bénéficier d'un meilleur suivi médical, notamment en ce qui concerne l'observance aux traitements; elle lui permet encore de simplifier la rédaction des fiches de renseignements médicaux, exigées dans de nombreuses circonstances de la vie courante - scolarité, centre d'hébergement, souscription d'un prêt, etc.

Pour le praticien, l'invention assure une complète transparence vis-à-vis du patient quant aux actes réalisés. Elle lui assure un accès simple et rapide à l'historique et à l'identité médicale du patient, avant d'établir un diagnostic; elle peut enfin lui permettre d'établir une communication avec le ou les praticiens ayant antérieurement suivi le patient.

L'invention assure encore un grande traçabilité des produits, et permet une étude statistique des consommations de santé. Elle permet aussi d'évaluer objectivement les prestations médicales fournies par les différents acteurs, de sorte à permettre un choix objectif par le patient.

Plus précisément, l'invention propose un serveur de données, comprenant pour un adhérent une partie protégée, la partie protégée pouvant être accédée :
- par l'adhérent en lecture d'une part et en écriture sans droit de modifications des données existantes d'autre part;
- pour une première classe d'utilisateurs en lecture et en écriture;
- pour une deuxième classe d'utilisateurs, en lecture uniquement des données non nominatives.

De préférence, le serveur comprend en outre une deuxième partie librement accessible.

Dans un mode de réalisation, on prévoit des moyens d'identification d'un utilisateur pour l'accès à la partie protégée.

De préférence, la partie protégée contient le dossier médical de l'adhérent. Elle peut comprendre une sous-partie active et une sous-partie historique. Dans ce cas, le passage des données de la partie active à la partie historique peut s'effectuer automatiquement en fonction du temps.

Avantageusement, la première classe d'utilisateurs comprend les médecins. En revanche, deuxième classe d'utilisateurs comprend les laboratoires pharmaceutiques.

Dans un mode de réalisation, le serveur présente des moyens de connexion à un réseau, notamment au réseau Internet.

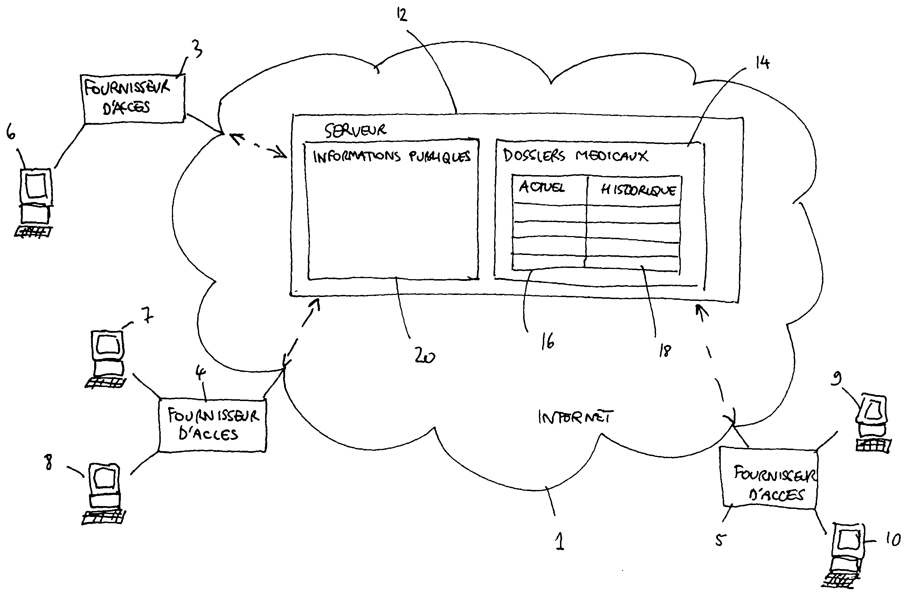

D'autres caractéristiques et avantages de l'invention apparaîtront à la lecture de la description qui suit de modes de réalisation de l'invention, donnée à titre d'exemple et en référence aux dessins annexés, dont la figure 1 montre une représentation schématique de l'invention.

L'invention propose de regrouper les données médicales d'un individu sur un serveur accessible à distance par l'intermédiaire d'un réseau tel que le réseau Internet; elle propose de ne permettre l'accès à ces données qu'à l'individu qu'elles concernent, et à son ou ses médecins. Pour l'individu, elle propose d'offrir un accès libre en lecture, mais limité en écriture à l'enrichissement des données, sans autorisation de modification; pour le ou les médecins, elle propose un accès libre en écriture et en lecture aux données des patients. Elle propose encore de permettre un accès limité et dans certains objectifs à d'autres que l'individu ou son médecin.

La figure 1 montre une représentation schématique du fonctionnement de l'invention. On a représenté à la figure 1 le réseau Internet 1, avec des fournisseurs d'accès 3, 4, 5 et les ordinateurs 6 à 10 d'utilisateurs. Ces utilisateurs sont typiquement des adhérents - personnes physiques -, des praticiens et des représentants de laboratoires ou centres hospitaliers - les acteurs du domaine de la santé - et des intervenants économiques, comme les assureurs.

La figure montre encore le serveur 12 selon l'invention. Celui-ci présente une première partie 14, qui est destinée à stocker le dossier médical complet de chaque personne ou famille, adhérent du service proposé. Ce dossier peut avantageusement être représenté sous une forme interactive, par exemple grâce à un corps humain dont les différentes parties sont susceptible d'être activées pour obtenir les informations correspondantes, ou sont munies d'un signe indiquant une pathologie ou maladie affectant cette ou ces parties. Le dossier comprend de préférence l'ensemble des interventions subies par l'adhérent, en particulier les produits médicaux ou chirurgicaux dont la traçabilité est intéressante : les prothèses pourront ainsi être référencés, par nom, marque, lot, chirurgien ayant réalisé l'intervention, lieu de l'intervention, etc.

Le dossier médical complet d'un individu peut aussi être un dossier dynamique, avec une sous-partie actuelle 16 contenant les traitements en cours, et une sous-partie historique 18 contenant les traitements passés; la fin d'un traitement peut alors provoquer son basculement automatique de la sous-partie actuelle vers la sous-partie historique.

Le caractère dynamique du dossier médical permet en outre d'émettre des rappels vers le patient, par courrier électronique, courrier ou autre. Pour préserver la confidentialité des informations, une transmission par courrier électronique peut être partiellement ou totalement codée en fonction de la confidentialité des informations proposées. Ces rappels peuvent concerner les rendez-vous de contrôle, les rappels de vaccination, la péremption des médicaments; de tels rappels peuvent être émis automatiquement par le serveur. On peut aussi prévoir d'émettre vers le patient des rappels manuels, par exemple en cas de suspicion d'une nouvelle pathologie par un praticien consultant le dossier médical.

Il est aussi possible de prévoir selon l'invention que cette première partie, pour un adhérent, soit traduite dans plusieurs langues, de sorte à permettre un suivi international du patient.

Selon l'invention, la première partie 12 du serveur n'est accessible que de façon limitée. Plus spécifiquement, l'accès d'un adhérent à son propre dossier médical est libre en lecture, dans la mesure où l'adhérent est dûment identifié. On peut employer à cette fin tous les moyens connus en soi, par exemple une carte personnelle d'adhérent et un code secret, permettant de limiter l'accès aux données de la première partie. En écriture, un adhérent a accès à son propre dossier médical, mais sans disposer du droit de modifier les données préexistantes dans le dossier. Il est donc possible pour un adhérent du service de compléter son dossier médical, si son médecin ne l'a pas fait, ou pour indiquer des traitements sans ordonnance. On peut notamment prévoir un menu interactif et contextuel qui guide l'adhérent et permet de contrôler la cohérence des données; ceci améliore la fiabilité des données qui peuvent être introduites par l'adhérent. On peut avantageusement prévoir que les modifications dans le dossier médical du patient sont assorties d'une mention de la date de modification et de l'auteur de la modification ainsi que de sa qualité.

L'invention propose encore que cette première partie soit libre d'accès par les praticiens, et plus spécifiquement par les praticiens soignant le patient. Ces praticiens constituent de ce point de vue une première classe d'utilisateurs. A cette fin, on peut encore utiliser les moyens connus en soi. Le praticien peut par exemple utiliser le code du patient, assorti de données personnelles du patient pour accéder aux informations du dossier du patient. On peut aussi fournir aux praticiens un code prioritaire permettant d'accéder au dossier médical indépendamment de l'état du patient, pour permettre un accès libre au dossier pour tous les membres du corps médical. Dans tous les cas, il est avantageux que les praticiens soient dûment identifiés, et on peut encore utiliser pour cela des moyens connus en soi, et par exemple fournir au praticien une carte.

L'invention propose encore que cette première partie du site soit accessible pour permettre l'extraction de données de façon anonyme, à une deuxième classe d'utilisateurs. Cette classe d'utilisateur peut comprendre les laboratoires, le gestionnaire du serveur, les pouvoirs publics, etc. Il est ainsi avantageux de permettre l'extraction des données concernant les matériaux utilisées pour les prothèses, les lots de médicaments, les durées de traitement pour un médicament donné, et toutes autres informations statistiques telles le comptage ou la localisation d'utilisation, qui sont utiles pour la recherche clinique. On peut aussi extraire de la première partie du site des liens entre les prothèses et les chirurgiens qui les ont posées. Ceci permet soit un suivi des prothèses et de leur prescription par les médecins; ceci peut aussi permettre de joindre plusieurs praticiens, par courrier électronique ou autres, pour établir un compte-rendu ou échanger des informations concernant un matériel commun.

Pour les laboratoires pouvant ainsi accéder aux informations de nature statistique, l'invention permet une traçabilité des produits, par la source directe du consommateur, un suivi des prescripteurs. On peut aussi prévoir la transmission d'information vers les adhérents utilisant un produit, sans que l'émetteur ne puisse les connaître; cette fonctionnalité permet un rappel des produits, une modification des notices, ou la transmission d'informations de toute nature, ciblées vers l'adhérent utilisateur de produits. Comme dans les cas précédents, on peut limiter l'accès des laboratoires aux informations, avec une identification appropriée, mise en oeuvre dans des moyens d'identification. Pour cela, on peut encore fournir à des employés d'un laboratoire une carte d'accès au serveur.

Dans la mesure où le serveur de l'invention est utilisé par les pharmaciens, on peut prévoir un suivi des produits prescrits, et notamment des génériques qui pourraient être substitués par le pharmacien.

Les cartes qui sont fournies aux adhérents pour l'accès à leur dossier médical, aux praticiens, ou aux laboratoires peuvent avantageusement comprendre un numéro d'identification du porteur de la carte. On peut prévoir un codage des droits attribués au porteur de la carte, par exemple en utilisant un code couleur représentatif de la catégorie d'utilisateur à laquelle appartient le porteur de la carte. On fournit en outre au porteur un code d'accès secret, qui permet le contrôle de son identification. Bien entendu, on peut aussi utiliser d'autres moyens d'identification et de contrôle, tels des cartes à puce.

L'invention propose encore que le serveur présente une seconde partie 20, d'un accès libre pour tous les usagers du réseau. Cette partie du site permet de fournir des données sur les structures médicales, publiques ou privées, par exemple par l'établissement d'un cahier des charges indiquant :
- la nature des prestations et services hôteliers et d'accueil des patients, assortie le cas échéant d'une indication de leur qualité;
- les moyens techniques dont disposent ces structures pour les soins prodigués aux patients, comme par exemple le personnel médical et paramédical, le personnel d'encadrement, le matériel, etc.

Cette partie du serveur peut aussi comprendre un forum de discussion permettant aux utilisateurs de cette seconde partie du serveur de donner leur opinion sur les prestations reçues, et donc de fournir un contrôle perpétuel des références.

Cette seconde partie du serveur permet de fournir une information fiable, complète et mise à jour sur l'ensemble des services médicaux; pour un assureur qui pourrait procéder à la mise en oeuvre du serveur, une telle information permet d'une part de se doter d'un outil de communication, mais aussi d'un outil d'analyse et de gestion des consommations de santé. Un tel outil permet un ajustement des contrats d'assurance santé, et notamment un remboursement fonction non pas d'un tarif unique, mais de la qualité des soins rendus, appréciés sur un base objective.

De ce point de vue, la carte fournie à l'utilisateur peut permettre un débit direct d'un compte de l'utilisateur auprès d'acteurs référencés par l'organisme - par exemple l'assureur - qui délivre la carte. L'invention fournit un système complet de suivi médical, qui va de la fourniture du dossier médical à la facturation des prestations - ou de la part non remboursable de celles-ci - en passant par le contrôle des médecins et des prescriptions.

Bien entendu, la présente invention n'est pas limitée aux exemples et modes de réalisation décrits et représentés, mais elle est susceptible de nombreuses variantes accessibles à l'homme de l'art. On pourrait ainsi utiliser un autre réseau que le réseau Internet pour l'accès au réseau. Le contenu des classes d'utilisateurs peut être modifié, en fonction des droits de chacun des intervenants.

## Revendications

1. Un serveur (12) de données, comprenant pour un adhérent une partie protégée (14), la partie protégée pouvant être accédée :
- par l'adhérent en lecture d'une part et en écriture sans droit de modifications des données existantes d'autre part;
- pour une première classe d'utilisateurs en lecture et en écriture;
- pour une deuxième classe d'utilisateurs, en lecture uniquement des données non nominatives.

2. Le serveur de la revendication 1, **caractérisé en ce qu'**il comprend en outre une deuxième partie (20) librement accessible.

3. Le serveur de la revendication 1 ou 2, **caractérisé par** des moyens d'identification d'un utilisateur pour l'accès à la partie protégée.

4. Le serveur de la revendication 1, 2 ou 3, **caractérisé en ce que** la partie protégée contient le dossier médical de l'adhérent.

5. Le serveur de l'une des revendications 1 à 4, **caractérisé en ce que** la partie protégée comprend une sous-partie active (16) et une sous-partie historique (18).

6. Le serveur de la revendication 5, **caractérisé en ce que** le passage des données de la partie active à la partie historique s'effectue automatiquement en fonction du temps.

7. Le serveur de l'une des revendications 1 à 6, **caractérisé en ce que** la première classe d'utilisateurs comprend les médecins.

8. Le serveur de l'une des revendications 1 à 7, **caractérisé en ce que** la deuxième classe d'utilisateurs comprend les laboratoires pharmaceutiques.

9. Le serveur de l'une des revendications précédentes, **caractérisé par** des moyens de connexion à un réseau, notamment au réseau Internet.
